# EUROPEAN PATENT APPLICATION

(11) **EP 1 134 290 A2**
(43) Date of publication of application: **19.09.2001**
(21) Application number: 01302116.7
(22) Date of filing: 08.03.2001
(51) Int. Cl.: C12Q 1/26, G06F 19/00, A61P 25/24

(54) **Pharmacophore models for the identification of the CYP2D6 inhibitory potency of selective serotonin reuptake inhibitors**

(30) Priority: 14.03.2000 US 189099 P
(71) Applicant: Pfizer Products Inc., Groton, Connecticut 06340 (US)
(72) Inventor: Ekins, Sean, Indianapolis, Indiana 46202 (US)
(74) Representative: Hayles, James Richard

(57) **Abstract**

The present invention relates to novel screening methods which enable the selection of selective serotonin reuptake inhibitor (SSRI) compounds which do not possess significant inhibitory potency towards cytochrome P450 enzymes, in particular, CYP2D6. The present invention also relates to a method of generating a pharmacophore model for the CYP2D6 inhibitory activity of SSRI compounds; to methods for the discovery of molecules that are potential SSRI compounds which do not possess significant inhibitory potency towards the CYP2D6 enzyme; to methods of modeling the features of the CYP2D6 pharmacophore useful in selecting SSRI's which do not possess significant potency towards CYP2D6. Further, the invention also relates to pharmaceutical compositions comprising an SSRI compound which does not possess significant potency towards the CYP2D6 enzyme identified by methods of the invention; to the uses of an SSRI compound identified by the methods of the invention for the manufacture of medicaments and for the treatment of a condition, a disorder or a disease in a mammal for which an SSRI compound identified by the method of the invention is therapeutically useful.

## Description

### BACKGROUND OF THE INVENTION

The present invention relates to novel screening methods which enable the selection of selective serotonin reuptake inhibitor compounds which do not possess significant inhibitory potency towards cytochrome P450 enzymes, in particular, CYP2D6. The present invention also relates to a method of generating a pharmacophore model of selective serotonin reuptake inhibitor compounds which do not possess significant inhibitory potency towards CYP2D6. The invention also relates to methods for the discovery of selective serotonin reuptake inhibitor compounds which do not possess significant inhibitory potency towards the CYP2D6 enzyme. The invention also relates to pharmaceutical compositions comprising a selective serotonin reuptake inhibitor compound that does not possess significant inhibitory potency towards CYP2D6 as identified by methods of the invention. The invention further relates to the uses of a selective serotonin reuptake inhibitor compound identified by the methods of the invention for the manufacture of medicaments and for the treatment of a condition, a disorder or a disease in a mammal for which such a selective serotonin reuptake inhibitor compound is therapeutically useful.

In the field of drug development, the developer of pharmaceutical substances must investigate the potential for clinically significant drug-drug interactions in the situations where more than one drug may be co-administered to a patient. There is also a significant need in the field of drug development to ascertain the potential existence of these drug-drug interactions prior to commencing any investigation into drug development. The identification of the strong potential usefulness of a chemical compound as early as possible *in vitro* saves considerable investment of time and resources.

The polymorphic enzyme, CYP2D6, represents only approximately 1.5% of the total human hepatic P450 (Shimada *et al*., *J. Pharmacol. & Exp. Pharmacol*., **270:** 414-423, 1994), yet participates in the metabolism of over 30% of clinically prescribed drugs (Lewis *et al*., *Xenobiotica*, **27**: 319-340, 1997) including many which have a narrow therapeutic index. (Spatzenegger and Jaeger, *Drug Metabolism Reviews, **27**:* 397-417, 1995; Wu *et al*., *Biochem. Pharmacol*., **53**: 1605-1612, 1997; Lewis *et al*., *supra*.). The clinical relevance of CYP2D6 is notable as approximately 7% of Caucasians are poor metabolizers of CYP2D6 substrates while 1% are ultrarapid metabolizers of CYP2D6 substrates. (Brosen, *Ther*. *Drug Monitoring,* **18:** 393-396, 1996). If a drug, which is an inhibitor of CYP2D6, is co-administered with a drug which has CYP2D6-mediated biotransformation as its major clearance mechanism, there is the potential danger of the occurrence of a clinically hazardous event depending on the concentration of the drugs at the enzymes in question.

Selective serotonin reuptake inhibitors (SSRI's) are a leading class of antidepressants. Since the introduction of fluoxetine in 1990 (Wong *et al*., *Life Sci.,* **57:** 411-441, 1995) and of numerous other similarly acting molecules since then, SSRI's have become in most cases the treatment of choice for depression and related illnesses. Selective serotonin reuptake inhibitor compounds are likely to be therapeutically useful in treating aggression disorders; anxiety disorders selected from the group consisting of panic attack, agoraphobia, panic disorder with or without agoraphobia, agoraphobia without history of panic disorder, specific phobia, social phobia, obsessive-compulsive disorder, post-traumatic stress disorder and acute stress disorder; cognitive disorders selected from the group consisting of amnestic disorders *(e.g.,* amnestic disorders due to a general medical condition, substance-induced persisting amnestic disorder and amnestic disorders otherwise not specified), deliriums *(e.g.,* deliriums due to a general medical condition, substance-induced delirium and delirium not otherwise specified), dementias *(e.g.,* dementia of the Alzheimer's type, vascular dementia, dementia due to a general medical condition *(e.g.,* AIDS-, Parkinson's-, head trauma-, and Huntington's-induced dementias), substance-induced persisting dementia, dementia due to multiple etiologies, and dementia otherwise not specified) and cognitive disorders otherwise not specified; depression disorders; emesis; epilepsy; food-related behavioral disorders, including anorexia nervosa and bulimia; headache disorders selected from the group consisting of migraine, cluster and vascular headaches; learning disorders, including attention deficit disorder and attention deficit/hyperactivity disorder; obesity; ocular disorders; platelet aggregation disorders; psychotic conditions selected from the group consisting of schizophrenia *(e.g.,* paranoid-type, disorganized-type, catatonic-type, undifferentiated-type and residual-type), schizophreniform disorder, schizoaffective disorder, delusional disorder, brief psychotic disorder, shared psychotic disorder, psychotic disorders due to a general medical condition and psychotic disorders otherwise not specified; sleep disorders selected from the group consisting of primary sleep disorders *(e.g.,* parasomnias and dyssomnias), sleep disorders related to another mental disorder (including, without limitation, mood and anxiety disorders), sleep disorders due to a general medical condition and sleep disorders otherwise not specified; sexual behavior disorders; substance-abuse disorders selected from the group consisting of alcohol-related disorders, including alcohol-use disorders *(e.g.,* dependence and abuse disorders) and alcohol-induced disorders *(e.g.,* intoxication, withdrawal, intoxication delirium, withdrawal delirium, persisting dementia, persisting amnestic, mood, anxiety, sexual dysfunction, sleep and disorders otherwise not specified), amphetamine-related disorders, including amphetamine-use disorders *(e.g.,* dependence and abuse disorders) and amphetamine-induced disorders *(e.g.,* intoxication, withdrawal, intoxication delirium, psychotic, mood, anxiety, sexual dysfunction, sleep and disorders otherwise not specified), caffeine-related disorders, such as intoxication, induced-anxiety disorder, induced-sleep disorder and disorders otherwise not specified; cannabis-related disorders, including cannabis-use disorders (*e.g*., abuse and dependence disorders) and cannabis-induced disorders *(e.g.,* intoxication, intoxication delirium, psychotic, anxiety and disorders otherwise not specified), cocaine-related disorders, including cocaine-use disorders *(e.g.,* dependence and abuse disorders) and cocaine-induced disorders *(e.g.,* intoxication, withdrawal, intoxication delirium, psychotic, mood, anxiety, sexual dysfunction, sleep and disorders otherwise not specified), hallucinogen-related disorders, including hallucinogen-use disorders *(e.g.,* dependence and abuse disorders) and hallucinogen-induced disorders *(e.g.,* intoxication, persisting perception, intoxication delirium, psychotic, mood, anxiety and disorders otherwise not specified), inhalant-related disorders, including inhalant-use disorders *(e.g.,* dependence and abuse disorders) and inhalant-induced disorders *(e.g.,* intoxication, intoxication delirium, persisting dementia, psychotic, mood, anxiety and disorders otherwise not specified), nicotine-related disorders, such as dependence, withdrawal and disorders otherwise not specified, opioid related disorders, including opioid-use disorders (*e.g*., dependence and abuse disorders) and opioid-induced disorders *(e.g.,* intoxication, withdrawal, intoxication delirium, psychotic, mood, sexual dysfunction, sleep and disorders otherwise not specified), phencyclidine-related disorders, including phencyclidine-use disorders (*e.g*., dependence and abuse disorders) and phencyclidine-induced disorders *(e.g.,* intoxication, intoxication delirium, psychotic, mood, anxiety and disorders otherwise not specified), sedative-, hypnotic- or anxiolytic-related disorders, including sedative-use disorders *(e.g.,* dependence and abuse disorders) and sedative-induced disorders *(e.g.,* intoxication, withdrawal, intoxication delirium, withdrawal delirium, persisting dementia, persisting amnestic, psychotic, mood, anxiety, sexual dysfunction, sleep and disorders otherwise not specified), polysubstance-related disorder, other substance dependence and abuse disorders, and other substance-induced disorders *(e.g.,* intoxication, withdrawal, delirium, persisting dementia, persisting amnestic, psychotic, mood, anxiety, sexual dysfunction, sleep and disorders otherwise not specified); vision disorders, including glaucoma; and, various additional diseases, disorders and conditions as well.

SSRI's have been documented to have drug-drug interaction issues to varying extents with CYP2D6 as well as other CYP's *in vivo* and *in vitro.* Many SSRI's are known to be inhibitors of cytochrome P450 (CYP), including CYP2D6 mediated metabolic pathways *in vivo* and *in vitro* (Belpair *et al*., *Eur. J. Clin. Pharmacol*., **54:** 261-264, 1998; Crewe *et al*., *Br. J. Clin. Pharmacol*., **34:** 262-265, 1992; Otton *et al., Clin. Pharmacol. Ther*., **53:** 401-409, 1993; Skjelbo *et al*., *Br. J. Clin. Pharmacol*., **34:** 256-261, 1992; Stevens *et al*., *J. Pharmacol. Exp. Ther*., **266:** 964-971, 1993). R-fluoxetine and R-norfluoxetine have been found to be more potent inhibitors of CYP2D6 than the S-isomers (Stevens *et al*., *supra*) which suggests that CYP2D6 exhibits some degree of stereoselectivity in terms of inhibition, possibly due to either access to or fitting into the active site. As CYP2D6 is of major concern because of its polymorphic nature (Brosen, *Ther*. *Drug Monitoring*, **18:** 393-396, 1996), the ability to predict whether a drug will interact with this CYP represents a serious advance in the pharmaceutical arts. The potential for CYP2D6 inhibition *in vivo* can be predicted reliably using systems such as human liver microsomes and recombinant enzymes, along with a know substrate probe for CYP2D6. By generating a K_{i (apparent)} value and knowledge of the plasma concentration of the drug, it is possible to reliably predict the likelihood of drug-drug interactions *in vivo* (Ring *et al*., *J. Pharmacol. Exp. Ther.,* **275**: 1131-1135, 1995).

There have been numerous reviews on the topic of drug-drug interactions and SSRI's which have focused on CYP2D6 (Baker *et al*., *Neurosci Biobehavioral Rev.,* **22:** 325-333, 1998; Brosen, *Int. Clin*. *Psychopharmacol*., **13** (suppl. 5): S45-S-47, 1998, Greenblatt *et al*., *J. Clin*. *Psychiatry*, **59** (suppl. 15): 19-27, 1998; Mitchell, *Drug Safety*, **17:** 390-406, 1997; Preskorn, *J*. *Psychopharmacol*., **12** (Suppl. B): S89-S97, 1998; Richelson, *J Clin. Psychiatry*, **59** (Suppl. 10): 22-26, 1998), as well as issues of chirality related to SSRI's (Baumann *et al*., *Int. Clin. Psychopharmacol*., **10** (Suppl. 1): 15-21, 1995; Lane *et al*., *Cell Mol. Neurobiol*., **19:** 355-372, 1999). Hence techniques to rapidly differentiate potential SSRI's (and their respective isomers, where appropriate) that are CYP2D6 inhibitors would be a powerful tool for future SSRI discovery and development.

Drug-drug interactions involving cytochrome P450 enzymes (CYPs), and in particular CYP2D6, are an important factor in the question of whether a new chemical entity will survive through to the development stage. CYPs are members of a large superfamily of heme-thiolate proteins involved in the metabolism of endobiotics and xenobiotics across eukaryotes and procaryotes. (Nelson *et al*., *Pharmacogenetics*, **6:** 1-42, 1996). The clinical relevance of CYPs are their central role in drug metabolism. They are present in all human tissues and may be inhibited by the co-administration of competing xenobiotics of the same enzyme. (Wrighton *et al*., *Toxicologic Pathology*, **23**: 199-208, 1995). Much less is known about the endogenous functions of CYPs, except for their role in steroid metabolism and recent postulations about their roles in neurotransmitter metabolism (Hiroi *et al*., *Biochem. Biophys. Res. Commun*., **249:** 838-43, 1998) and signaling pathways (Chan *et al*., *Proc. Natl. Acad. Sci.,* **95:** 10459-10464, 1998). The current understanding of the structural requirements of the CYP active site are presently limited to homology models using P450_{CAM}, P450_{TERP} and P450_{eryF}. (Lewis *et al*., *supra*).

Therapeutic drug monitoring of CYP2D6 modulators is costly and impacts on health care costs; thus minimizing interactions with CYP2D6 is advantageous to the patient and the entire health care system. Ultimately, knowledge of CYP2D6 inhibitory potential may impact on whether a drug will be co-administered with drugs that are known substrates of CYP2D6. Accordingly, the ability to predict the likelihood of a molecule being a CYP2D6 inhibitor early in the discovery process allows for the more efficient synthesis of suitable candidate molecules without the structural features that cause undesirable inhibition. A means of screening for drugs that do not have significant interactions with the CYP2D6 enzyme is therefore desirable.

Present technologies have centered around the use of *in vitro* testing using human liver microsomes or recombinant CYPs and a known catalytic probe for CYP2D6 such as bufuralol. However, the possible volume of *in vitro* studies is often limited by equipment and materials cost, incubation volume and the rate of analytical determination. Alternatives to *in vitro* techniques as a preliminary screen to enable the selection of compounds for later study *in vitro* would need to be fast, cost-effective and reliable. Numerous studies have described the variability of K_{i (apparent)} values for serotonin reuptake inhibitors (SSRI's) against CYP2D6. Generating this *in vitro* parameter appears to be dependent on the substrate probe used as well as other experimental variables.

One such alternative, embraced by the present invention, is the use of computational quantitative structure activity relationship (QSAR) modeling techniques as a screening device for inhibitory potency towards CYP2D6. In the past, computational techniques have led to the production of some computer-generated substrate templates, pharmacophores as well as homology models of the active site of CYPs. A pharmacophore model generated by SYBYL has been derived from CYP2D6 inhibitors of bufuralol 1'-hydroxylation as a selective probe for generating Kᵢ (inhibition constant) values. (Strobl *et al*., *J. Med. Chem.,* **36:**1136-1145, 1993). This inhibitor pharmacophore model suggested that a positive charge on a nitrogen atom and a flat hydrophobic region extending to 7.5 Å virtually perpendicular along the N-H axis are requirements for inhibitory activity. (Strobl *et al*., *Id*.). Very recently, attempts at pharmacophore modeling of diverse inhibitors using CATALYST™ have been described which were used prospectively or retrospectively to predict inhibitor binding affinity for CYP2D6 (Ekins *et al*., *Pharmacogenetics*, **9:** 477-489, 1999).

To date, however, closely related molecules from a single therapeutic class have not been used to model the CYP2D6 active site from the point of view of inhibitory activity. There have only been QSAR models from other CYPs generated using a single therapeutic class of compounds, such as quinolones for CYP1A2 (Fuhr *et al*., *Mol. Pharmacol*., **43:** 191-199, 1993) or warfarin analogs for CYP2C9 (Jones *et al*., *Drug Metabolism & Disposition*, **24**: 1-6, 1996).

Approaches towards modeling the common features of substrates and inhibitors of human CYPs in general from data generated *in vitro* have been recently shown using a CATALYST™ pharmacophore approach (Ekins *et al*., *supra*; Ekins *et al*., *J. Pharmacol. & Exp. Ther*., **288:** 21-29, 1999; Ekins *et* al., *J. Pharmacol. & Exp. Ther*., **290:** 429-438, 1999; Ekins *et al*., *J. Pharmacol. & Exp*. *Ther*., **291:** 424-433, 1999), comparative molecular field analysis (CoMFA) (Jones *et al*., *supra*), and a molecular descriptor method (Bravi and Wikel, in press, *Quant. Struct. Act. Rel.,* 2000). Two of these 3D-quantitative structure activity relationship (3D-QSAR) techniques have also been used to predict a test set of molecules absent from the training set (Ekins *et al*., *J. Pharmacol. & Exp. Ther.,* **288:**21-29 (1999)). The utility of predictive CYP2D6 inhibitor pharmacophores had been recently shown in comparison to K_{i (apparent)} data (Ekins *et al*., *Pharmacogenetics*, **9:** 477-489, 1999) where models generated with the CATALYST™ program exhibited reasonable success in the prediction of K_{i (apparent)} values for molecules in a test set, outside of the training set.

A more recent technique is an *in silico* pharmacophore which describes the three dimensional chemical features necessary for inhibition (Ekins *et al*., *Pharmacogenetics*, ***9:*** 477-489, 1999). The first CYP2D6 pharmacophore was capable of being used in a semiquantitative manner (Strobl *et al*., *J Med. Chem*., ***36:*** 1136-1145 (1993)). More recently, this same data set was used along with a second pharmacophore as the first attempts at truly quantitative and reasonably predictive computational models to predict differing classes of CYP2D6 inhibitors obtained from the literature (Ekins *et al*., *Pharmacogenetics*, ***9**:* 477-489, 1999). The test sets for the previously described pharmacophores contained six SSRI's (fluvoxamine, paroxetine, sertraline, citalopram, desmethylcitalopram, and desmethylsertraline) out of which 5 of the 6 were predicted to within 1 log of the mean K_{i (apparent)}, obtained from numerous literature sources (Ekins *et al*., *Pharmacogenetics*, *9:* 477-489, 1999). This success is not too surprising as the pharmacophore contained both isomers of fluoxetine and norfluoxetine.

The present invention however makes it possible to obtain a CYP2D6 pharmacophore from a diverse library of SSRI's, some of which are not well known. This can then be used to predict reliably the K_{i (apparent)}values of SSRI's and correctly rank by potency the isomers of the SSRI's, including, *e.g*., fluoxetine and norfluoxetine. The pharmacophore model of the present invention for the CYP2D6 inhibitory activity of SSRI's makes possible the screening, discovery, selection and design of molecules that possess the selective serotinin reuptake activity without having undesired interactions with the CYP2D6 enzyme.

### SUMMARY OF THE INVENTION

The present invention is directed to a method of generating a pharmacophore model for the CYP2D6 inhibitory potency of SSRI compounds comprising the steps of
(i) generating a set of three-dimensional conformers for each of the compounds in a training set comprising five or more SSRI compounds;
(ii) correlating each of the compounds of said training set with an observed value for CYP2D6 inhibitory potency;
(iii) generating from the conformers generated in step (i) a set of one or more pharmacophore test models, each said pharmacophore test model comprising three or more CYP2D6 enzyme active site features selected from the group consisting of the hydrogen bond donor feature, the hydrogen bond acceptor feature, the hydrophobic region feature, the ionizable region feature and the ring aromatic feature, arranged in three-dimensional space;
(iv) calculating the CYP2D6 inhibitory potency for each conformer generated in step (i) towards each of the pharmacophore test models generated in step (iii);
(v) calculating the total cost (or goodness of fit) for each pharmacophore test model; and
(vi) choosing the lowest cost (or best fit) pharmacophore test model as the pharmacophore model.

Preferably, each of the steps of the methods of the invention are carried out using molecular modeling software, more preferably one such as CATALYST™, version 4 (Molecular Simulations, Inc., San Diego, CA), or other modeling programs known to those of skill in the art.

The term "training set," as used herein, refers to the set of compounds used to build the pharmacophore model that possess known CYP2D6 inhibitory activity. The training set of SSRI compounds in step (i) are preferably chosen from known SSRI compounds with CYP2D6 inhibitory activity values which span at least several orders of magnitude, more preferably a K_{i (apparent)} of approximately 0.1 µM to 100 µM. One preferred method uses the training set of 14 amphetamine compounds referred to in Wu *et al*., *Biochem. Pharmacol*., **53:** 1605-1612 (1997) (observed K_{i (apparent)} values in µM in parentheses):
(*d*,*l*)-2-methoxy-4,5-methylenedioxyamphetamine (0.17);
(*d*,*l*)-3,4-methylenedioxymethamphetamine (0.6);
(*d*,*l*)-3,4-methylenedioxyamphetamine (1.8);
(*d*,*l*)-3-methoxy-4,5-methylenedioxyamphetamine (2.2);
(*d*,*l*)-2-methoxyamphetamine (11.5);
(*d*,*l*)-3-methoxyamphetamine (17.5);
(*d*,*l*)-4-methoxyamphetamine (24);
(+)-methamphetamine (25);
(+)-amphetamine (26.5);
(*d*,*l*)-2,4,6-trimethoxyamphetamine (33);
(*d*,*l*)-4-hydroxymethamphetamine (60);
(*d*,*l*)-3,4,5-trimethoxyamphetamine (128);
(+)-4-hydroxyamphetamine (195); and
(*d*,*l*)-cathinone (340).

Further, the number of conformers in step (i) is preferably limited to 175 conformers, more preferably 255 conformers, with a potential energy range of 50 Kcal/mole, preferably 35 Kcal/mole, most preferably 10 Kcal/mole.

The term "pharmacophore test model" as used herein, refers to a best guess (whether random or based upon a composite skewed in favor of the compounds in the training set exhibiting a high degree of CYP2D6 inhibitory potency) for the three-dimensional orientation of a set of features which describe the physical, chemical and/or electronic environment of active site of the CYP2D6 enzyme, said features comprising, *e.g*., the hydrogen bond donor feature, the hydrogen bond acceptor feature, the hydrophobic region feature, the ionizable region feature and the ring aromatic feature. Preferably, in step (iii), at least ten pharmacophore test models are generated.

In step (iv), the calculation of the CYP2D6 inhibitory potency for each of the conformers of the compounds towards a particular pharmacophore test model is preferably performed via a "fast-fit" algorithm which finds the optimum fit of the particular conformer of a compound to a particular pharmacophore test model without performing an energy minimization on the conformers of the compound. The calculated CYP2D6 inhibitory potency may alternatively be calculated by means of a linear regression equation, among other techniques, which correlates the input parameters, *i.e.,* the observed CYP2D6 inhibitory potency of a particular compound, with the features present in that compound. Solving the equation for a particular conformer in a particular pharmacophore test model yields a calculated inhibitory potency value. Preferably the CYP2D6 inhibitory potency is based upon observed potency values, preferably K_{i (apparent)} values, or alternatively, IC₅₀ values.

The total "cost" (or goodness of fit) for each pharmacophore test model in step (v) is calculated from the deviation between the estimated CYP2D6 inhibitory activity and the observed activity for each compound combined with the number of pharmacophore features in the pharmacophore test model. The pharmacophore test model with the lowest "cost" (or deviation between estimated and observed activity) is the model that is selected as it generally possesses features representative of all of the generated pharmacophore test models. Preferably, the observed CYP2D6 activity is given by the K_{i (apparent)} values measured *in vitro.* Alternatively, the IC₅₀ values measured *in vitro* of the inhibition of recombinant CYP2D6 enzyme activity in the presence of a known substrate, such as in the bufuralol l'hydroxylase assay, may also be used as indicators of observed activity.

The present invention also relates to a method for screening an SSRI compound for significant inhibitory potency towards CYP2D6 comprising the steps of
(i) finding the optimum fit of the SSRI compound to the pharmacophore model of the present invention; and
(ii) calculating the CYP2D6 inhibitory potency for the SSRI compound.

Another preferred method of the present invention for generating a pharmacophore model for the CYP2D6 inhibitory potency of SSRI compounds comprises the step of
(i) correlating the chemical features of a training set of SSRI compound conformers with a set of two- and/or three-dimensional descriptors for the active site of the CYP2D6 enzyme; and
(ii) generating an equation relating the observed CYP2D6 inhibitory potency of the training set of SSRI compounds to a set of generated two- and/or three-dimensional descriptors for the SSRI compound.

The pharmacophore model in this instance is in the form of an equation. Preferably, each of the steps of the methods of the invention are carried out using molecular modeling software, more preferably, one such as CERIUS² ™ (Molecular Simulations, Inc., San Diego, CA), or other modeling programs known to those of skill in the art.

Preferably, the steps of this method may be carried out using the set of two-and/or three-dimensional descriptors for a compound molecule found in the 3D-QSAR functionality of CERIUS² ™. Step (ii) of the method is preferably carried out using a genetic function approximation (GFA) equation. However, one may also use principle component analysis or partial least squares to correlate descriptors and activity. In addition, there are other regression techniques (linear or non-linear) available and known to those of skill in the art to perform this correlation.

The present invention is also directed to a method for screening SSRI compounds for CYP2D6 inhibitory potency comprising the steps of
(i) generating the two- and/or three-dimensional descriptors for an SSRI compound;
(ii) inputting said two- and/or three-dimensional descriptors into a pharmacophore model equation relating the measured CYP2D6 inhibitory potency of a training set of SSRI compounds to a set of two- and/or three-dimensional descriptors generated for those SSRI compounds; and
(iii) solving said equation for the CYP2D6 inhibitory activity of the SSRI compound corresponding to the generated three-dimensional descriptors of step (i).

Steps (i) through (iii) are preferably carried out using a software program, *e.g*., CERIUS² ™, among others, known to those of skill in the art.

The present invention is also directed to a pharmacophore model for the CYP2D6 inhibitory potency of SSRI compounds generated in accordance with the methods of the present invention. The pharmacophore model of the invention is preferably three-dimensional and comprises a set of features, each of which is defined by Cartesian coordinates, x, y and z, which represent the centroid of the feature, and a vector for each feature originating from the centroid of the feature, the direction of which vector is also defined by Cartesian coordinates. The vector represents the optimal directionality of the feature, *e.g*., the direction of an optimal hydrogen bond for hydrogen-bonding features, *inter alia*. Preferably, the model comprises at least three features: 1 hydrogen bond acceptor, 1 hydrogen bond donor and 1 hydrophobic feature.

The coordinates of the model of the invention defines the relative relationship between the features, and therefore, those of skill in the art will recognize that the specific coordinates are dependent upon the specific coordinate system used, and thus, although rotation or translation of these coordinates may change the specific values of the x, y and z coordinates, the coordinates will define the claimed model. Those skilled in the art will also recognize that the model of the invention may encompass any model, after optimal superposition of the molecules, comprising the identified features and having a root mean square of equivalent features of less than about 3.0 Å. More preferably, the model of the invention encompasses any model comprising the identified features and having a root mean square of equivalent features of less than about 1.5 Å, and most preferably, less than about 1.0 Å.

The present invention also relates to a method for identifying SSRI compounds that do not possess significant inhibitor potency towards CYP2D6 from structural and literature databases of experimental compounds comprising the use of the pharmacophore model generated in accordance with the methods of the present invention. In a preferred embodiment, an SSRI antagonist compound that does not possess significant inhibitory potency towards CYP2D6 is identified by predicting the K_{i (apparent)} value (or alternatively, the IC₅₀ value) of said compound. Preferably, the Kᵢ ₍ₐₚₚₐᵣₑₙₜ₎ value for an SSRI compound that does not possess significant inhibitory potency towards CYP2D6 is greater than or equal to 1 µM; more preferably, greater than or equal to 10 µM; and most preferably, greater than or equal to 100 µM.

The present invention also relates to SSRI compounds that do not possess significant inhibitory potency towards CYP2D6 identified by the methods of the present invention. Further, the present invention is directed to pharmaceutical compositions comprising an SSRI compound that does not possess significant inhibitory potency towards CYP2D6 identified by the methods of the present invention.

The present invention further relates to methods of treatment for a condition, disorder or disease for which an SSRI compound is therapeutically useful comprising the administration of an SSRI compound that does not possess significant potency towards CYP2D6, a pharmaceutically acceptable derivative or pharmaceutical composition thereof, identified by a method of the present invention comprising the use of the pharmacophore model of the invention. The condition, disease or disorder for which an SSRI compound is therapeutically useful may be selected from the group consisting of treating aggression disorders; anxiety disorders selected from the group consisting of panic attack, agoraphobia, panic disorder with or without agoraphobia, agoraphobia without history of panic disorder, specific phobia, social phobia, obsessive-compulsive disorder, post-traumatic stress disorder and acute stress disorder; cognitive disorders selected from the group consisting of amnestic disorders *(e.g.,* amnestic disorders due to a general medical condition, substance-induced persisting amnestic disorder and amnestic disorders otherwise not specified), deliriums *(e.g.,* deliriums due to a general medical condition, substance-induced delirium and delirium not otherwise specified), dementias (*e.g*., dementia of the Alzheimer's type, vascular dementia, dementia due to a general medical condition *(e.g.,* AIDS-, Parkinson's-, head trauma-, and Huntington's-induced dementias), substance-induced persisting dementia, dementia due to multiple etiologies, and dementia not otherwise specified) and cognitive disorders otherwise not specified; depression disorders; emesis; epilepsy; food-related behavioral disorders, including anorexia nervosa and bulimia; headache disorders selected from the group consisting of migraine, cluster and vascular headaches; learning disorders, including attention deficit disorder and attention deficit/hyperactivity disorder; obesity; ocular disorders; platelet aggregation disorders; psychotic conditions selected from the group consisting of schizophrenia *(e.g.,* paranoid-type, disorganized-type, catatonic-type, undifferentiated-type and residual-type), schizophreniform disorder, schizoaffective disorder, delusional disorder, brief psychotic disorder, shared psychotic disorder, psychotic disorders due to a general medical condition and psychotic disorders otherwise not specified; sleep disorders selected from the group consisting of primary sleep disorders (*e.g*., parasomnias and dyssomnias), sleep disorders related to another mental disorder (including, without limitation, mood and anxiety disorders), sleep disorders due to a general medical condition and sleep disorders otherwise not specified; sexual behavior disorders; substance-abuse disorders selected from the group consisting of alcohol-related disorders, including alcohol-use disorders *(e.g.,* dependence and abuse disorders) and alcohol-induced disorders *(e.g.,* intoxication, withdrawal, intoxication delirium, withdrawal delirium, persisting dementia, persisting amnestic, mood, anxiety, sexual dysfunction, sleep and disorders otherwise not specified), amphetamine-related disorders, including amphetamine-use disorders *(e.g.,* dependence and abuse disorders) and amphetamine-induced disorders *(e.g.,* intoxication, withdrawal, intoxication delirium, psychotic, mood, anxiety, sexual dysfunction, sleep and disorders otherwise not specified), caffeine-related disorders, such as intoxication, induced-anxiety disorder, induced-sleep disorder and disorders otherwise not specified; cannabis-related disorders, including cannabis-use disorders *(e.g.,* abuse and dependence disorders) and cannabis-induced disorders *(e.g.,* intoxication, intoxication delirium, psychotic, anxiety and disorders otherwise not specified), cocaine-related disorders, including cocaine-use disorders *(e.g.,* dependence and abuse disorders) and cocaine-induced disorders *(e.g.,* intoxication, withdrawal, intoxication delirium, psychotic, mood, anxiety, sexual dysfunction, sleep and disorders otherwise not specified), hallucinogen-related disorders, including hallucinogen-use disorders *(e.g.,* dependence and abuse disorders) and hallucinogen-induced disorders *(e.g.,* intoxication, persisting perception, intoxication delirium, psychotic, mood, anxiety and disorders otherwise not specified), inhalant-related disorders, including inhalant-use disorders *(e.g.,* dependence and abuse disorders) and inhalant-induced disorders *(e.g.,* intoxication, intoxication delirium, persisting dementia, psychotic, mood, anxiety and disorders otherwise not specified), nicotine-related disorders, such as dependence, withdrawal and disorders otherwise not specified, opioid-related disorders, including opioid-use disorders *(e.g.,* dependence and abuse disorders) and opioid-induced disorders *(e.g.,* intoxication, withdrawal, intoxication delirium, psychotic, mood, sexual dysfunction, sleep and disorders otherwise not specified), phencyclidine-related disorders, including phencyclidine-use disorders *(e.g.,* dependence and abuse disorders) and phencyclidine-induced disorders *(e.g.,* intoxication, intoxication delirium, psychotic, mood, anxiety and disorders otherwise not specified), sedative-, hypnotic- or anxiolytic-related disorders, including sedative-use disorders *(e.g.,* dependence and abuse disorders) and sedative-induced disorders *(e.g.,* intoxication, withdrawal, intoxication delirium, withdrawal delirium, persisting dementia, persisting amnestic, psychotic, mood, anxiety, sexual dysfunction, sleep and disorders otherwise not specified), polysubstance-related disorder, other substance dependence and abuse disorders, and other substance-induced disorders *(e.g.,* intoxication, withdrawal, delirium, persisting dementia, persisting amnestic, psychotic, mood, anxiety, sexual dysfunction, sleep and disorders otherwise not specified); vision disorders, including glaucoma; and, various additional diseases, disorders and conditions as well. Further, the invention also relates to pharmaceutical compositions comprising an SSRI compound identified by the methods of the invention.

The present invention is also related to a method of designing *de novo* compounds that are SSRI compounds that do not possess significant inhibitory potency towards CYP2D6 comprising the step of (i) correlating the two- and/or three-dimensional descriptors for a pharmacophore model for SSRI compounds that possess significant inhibitory potency towards CYP2D6 with randomly generated molecules having chemical features corresponding to said descriptors; and (ii) choosing a generated molecule with a CYP2D6 K_{i (apparent)} of 1 µM or greater. Preferably, the CYP2D6 inhibitory activity should correspond to an K_{i (apparent)} of 10 µM or greater; more preferably, 100 µM or greater. The compounds having features corresponding to said descriptors may be randomly generated by any variety of computational methods from a library of known chemical features and conformational preferences of chemical groups and multiple chemical groupings.

The present invention is also related to a method of designing *de novo* SSRI compounds which have selective inhibitory potency towards CYP2D6 comprising the step of
(i) choosing a target degree of CYP2D6 inhibitory potency;
(ii) generating a set of two- and/or three-dimensional descriptors for a pharmacophore model for SSRI compounds that possess significant inhibitory potency towards CYP2D6 corresponding to the inhibitory potency of step (i); and
(iii) correlating said descriptors of step (ii) with compounds having chemical features corresponding to said descriptors.

The present invention relates to a computer-readable medium having stored thereon a pharmacophore model for the inhibitory potency towards CYP2D6 of SSRI compounds generated in accordance with the methods of the present invention. The present invention also encompasses computers comprising such a computer-readable medium. The present invention relates to a computer comprising a pharmacophore model for CYP2D6 inhibitory potency of SSRI compounds for use in the design or screening of a molecular structure having SSRI activity and CYP2D6 inhibitory activity. A further embodiment of the invention is the combination of this CYP2D6 inhibition pharmacophore model with a pharmacophore model for SSRI biological activity.

The term "significant inhibitory potency" in the context of enzyme inhibition, unless otherwise indicated, refers to the ability of a compound over a characteristic concentration range to interfere with the function of said enzyme, whether permanently or temporarily, so as to deprive said enzyme of the ability to effect or participate in the transformation of chemical and/or biological substances.

The term "treating" refers to, and includes, reversing, alleviating, inhibiting the progress of, or preventing a disease, disorder or condition, or one or more symptoms thereof; and "treatment" and "therapeutically" refer to the act of treating, as defined above.

The term "chemical dependency," as used herein, means an abnormal craving or desire for, or an addiction to a drug. Such drugs are generally administered to the affected individual by any of a variety of means of administration, including oral, parenteral, nasal or by inhalation. Examples of chemical dependencies treatable by the methods of the present invention are dependencies on alcohol, nicotine, cocaine, heroin, phenobarbital, and benzodiazepines (*e.g.*, Valium™); "treating a chemical dependency," as used herein, means reducing or alleviating such dependency.

### BRIEF DESCRIPTION OF THE FIGURE

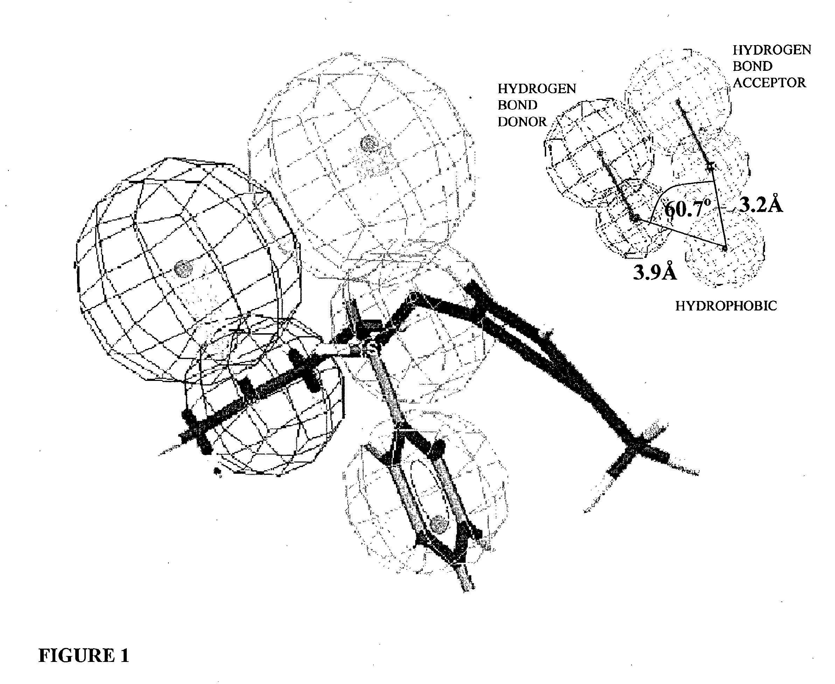

Figure 1: S-fluoxetine fitted to the CYP2D6 K_{i (apparent)} pharmacophore from the data of Wu *et al*., *supra*. The pharmacophore contains a hydrophobic area, a hydrogen bond acceptor and a hydrogen bond donor. The latter two features have vectors in the direction of the putative hydrogen bond donor and receptors, respectively in the active site. Inset: the interbond angles and distances between the labeled pharmacophore features.

### DETAILED DESCRIPTION OF THE INVENTION

The present invention permits the prediction of the CYP2D6 inhibitory potency of SSRI compounds. The methods of the present invention generate various quantitative structure activity relationship (QSAR) models (or pharmacophore models) using *in vitro* data for the ability of certain SSRI values compounds to inhibit the activity of recombinant CYP2D6. In particular, in one embodiment, the present invention comprises a computational method which uses previously known K_{i (apparent)} values for amphetamine analogues to build the pharmacophore model. Although *in vitro* IC₅₀ values are also often used to assess inhibitory potency, the use of any indicator of CYP2D6 inhibitory potency in the methods of the invention affords the opportunity to build a comprehensive structure activity relationship around an individual CYP.

SSRI compounds were used to create training sets for molecular modeling using CATALYST™. This software uses a collection of molecules with CYP2D6 inhibitory potency over multiple orders of magnitude for the enzyme of interest, in order to construct a model of the structural features (pharmacophore) necessary for the interaction of molecules with the active site of the enzyme. The resultant pharmacophore test models explain the variability of the potency of inhibition with respect to the geometric localization of these features of molecules.

The computational models constructed by the present invention utilize training sets of SSRI antagonists with observed CYP2D6 K_{i (apparent)} values varying over several orders of magnitude. The specific compounds utilized in conjunction with the exemplified models are shown in Table I along with observed CYP2D6 K_{i (apparent)} values. Nonetheless, the models and methods of the invention are not limited to the use of these particular compounds but encompass the use of other SSRI's known to those of skill in the art.

**Table I.**

| **Observed and Predicted Ki (apparent) Values for Inhibitors of CYP2D6 Fit to the CATALYST™ Pharmacophore** | | |
|---|---|---|
| SSRI | Observed K_{i (apparent)} (µM) | Predicted^{a} K_{i (apparent)} (µM) |
| Sertraline | 1.2^{b} | 46 (0.84) |
| Sertraline | 1.5^{c} | |
| Sertraline | 22.7^{d} | |
| Sertraline | 0.7^{e} | |
| Desmethylsertraline | 16^{d} | 46 (0.45) |
| Fluoxetine | 0.67^{f} | 0.8 (-0.13) |

| SSRI | Observed K_{i (apparent)} (µM) | Predicted^{a} K_{i (apparent)} (µM) |
|---|---|---|
| Fluoxetine | 0.92^{g} | |
| Fluoxetine | 0.6^{e} | |
| Fluoxetine | 0.17^{b} | |
| Fluoxetine | 3^{d} | |
| S-Fluoxetine | 1.38^{h} | 1.0(0.54) |
| R-Fluoxetine | 0.22^{h} | 0.77 (-0.23) |
| Norfluoxetine | 0.91^{f} | 0.62 (-0.31) |
| Norfluoxetine | 0.33^{g} | |
| Norfluoxetine | 0.43^{e} | |
| Norfluoxetine | 0.19^{b} | |
| Norfluoxetine | 3.5^{d} | |
| S-Norfluoxetine | 1.48^{h} | 0.72 (-0.31) |
| R-Norfluoxetine | 0.31^{h} | 0.65 (0.32) |
| Citalopram | 5.1^{e} | 400 (1.59) |
| Citalopram | 19^{g} | |
| Citalopram | 7^{b} | |
| Desmethylcitalopram | 1.3^{g} | 1.2 (-0.48) |
| Desmethylcitalopram | 6^{b} | |
| Paroxetine | 0.065^{c} | 1.2 (0.80) |
| Paroxetine | 0.15^{e} | |
| Paroxetine | 0.36^{g} | |
| Fluvoxamine | 0.38^{f} | 0.44 (-1.27) |
| Fluvoxamine | 8.2^{e} | |
| Fluvoxamine | 3.9^{g} | |
| Fluvoxamine | 26.6ⁱ | |

| SSRI | Observed K_{i (apparent)} (µM) | Predicted^{a} K_{i (apparent)} (µM) |
|---|---|---|
| Fluvoxamine | 1.8^{c} | |

| | | |
|---|---|---|
| ^{a} Values in parentheses represent residual (log units) of mean predicted minus observed K_{i (apparent)} values. | | |
| ^{b} Otton *et al*., *supra*. | | |
| ^{c} Otton *et al*., *Clin. Pharmacol. Ther*., **55:** 141, 1994. | | |
| ^{d} von Moltke *et al*., *J. Pharmacol. Exp. Ther*., **268:** 1278-1283, 1994. | | |
| ^{e} Crewe *et al*., *supra*. | | |
| ^{f} Belpaire *et al*., *supra*. | | |
| ^{g} Skjelbo *et al*., *supra*. | | |
| ^{h} Stevens *et al*., *supra.* | | |
| ⁱ von Moltke *et al*., *J. Clin. Psychopharmacol*., **15**: 125-131, 1995. | | |

The model of the three dimensional structure activity relationships (3D-QSAR) of the common structural features of the CYP2D6 inhibitor compounds is built, as described herein, using the CATALYST™ program. In addition, a QSAR two-and/or three-dimensional descriptor-based models may also be constructed with the CERIUS² ™ program. As those of skill in the art will readily recognize, chemically different substructures can present certain identical three-dimensional space-filling features, and accordingly, the models of the present invention comprise features which may or may not correspond to actual functional groups in any given SSRI compound.

In connection with the present invention, SSRI's identified as displaying inhibitory potency towards CYP2D6 were used. CATALYST™ models suggest that hydrophobic, hydrogen bond acceptor and hydrogen bond donor features are necessary to describe CYP2D6 inhibitory activity. The use of another modeling technique using the CERIUS² ™ program, which although less visual than CATALYST™, can generate multiple descriptors for molecules and via the use of more physicochemical parameters. This is important, as such parameters are useful in determining CYP inhibition and selectivity.

The validity of the predictive nature of the CATALYST™ model developed using 14 amphetamine compounds of Wu *et al.* was evaluated using a test set of SSRI compounds not present in this training set. This model and others generated in accordance with the methods of the invention enable database screening for CYP2D6 inhibition.

The CATALYST™ model was generated from multiple conformers of the 14 amphetamine compounds of Wu *et al*. and consisted of 3 pharmacophoric features and demonstrated a correlation of observed and predicted K_{i (apparent)} values (r = 0.87). The CATALYST™ model predicted 10 out of the 12 molecules well using a 1 log unit residual cutoff as previously described (Ekins *et al*., *J*. *Pharmacol. Exp. Ther*., **288**: 21-29, 1999), with only fluvoxamine and citalopram failing. The deviation in these comparisons may dependent on the fact that a mean of the published data for each compound is used. The published data is in most cases is quite variable, *e.g.,* fluoxetine, norfluoxetine, sertraline, paroxetine and fluvoxamine K_{i (apparent)} values vary by > 1 log unit (Table I).

In a previous pharmacophore study with a test set containing 6 SSRI's, paroxetine and desmethylcitalopram were poorly predicted (Ekins *et al*., *Pharmacogenetics*, **9:** 477-489, 1999). The model of the present invention however predicts SSRI's very well, and is able to distinguish and correctly the rank the CYP2D6 inhibitory potency for the stereoisomers of fluoxetine and norfluoxetine as obtained *in vitro* (Stevens *et al*., *supra*). This capability of the pharmacophore model of the invention makes possible the virtual design of future SSRI's as it suggests it will allow selection of the least potent isomer of a molecule for inhibition of CYP2D6.

A CATALYST™ pharmacophore model generated in accordance with the methods of the invention has the features as set forth in Figure 1. As depicted, the model comprises a set of features which are arranged in three-dimensional space and describe a chemical property or characteristic attributable to certain arrangements of atoms within molecules. In general, these features, *e.g*., a hydrogen bond acceptor, may be defined as any nitrogen, oxygen or sulfur atom with at least one available *(e.g.,* non-delocalized) lone electron pair; a hydrogen bond donor, may be defined by the availability of an electropositive hydrogen atom; a ring aromatic feature, may be a hydrophobic, planar feature *(e.g.,* phenyl, *etc.).* Complete definitions of these individual features have been described elsewhere and will be understood by those of skill in the art. (Greene *et al*., *J. Chem. Inf. & Comp. Sci*., **34:** 1297-1308, 1994). Cartesian coordinates that are displacements in Ångstroms along x, y and z axes define the centroid of each feature of the pharmacophore model. Furthermore, each feature normally has an optimal directionality defined by a vector, which originates (tail) from the centroid of the feature and ends (head) in the coordinates provided in the model. For example, for the hydrogen bond acceptor, the model contains the optimal direction for the formation of a hydrogen bond between the feature of the model and a chemical group in the active site of CYP2D6; for the ring aromatic feature, the vector would be perpendicular to the planes of the aromatic ring and defines the rotation of these features for optimal fit into the active site.

**Table II.**

| **Summary of features and positions for CATALYST™ model based on the data of Wu et al. (1997) used to predict CYP2D6 Inhibition by SSRI's.** | | | | | |
|---|---|---|---|---|---|
| **Coordinates** | **Hydrogen Bond Acceptor** | | **Hydrogen Bond Donor** | | **Hydrophobic** |
| | | **Vector** | | **Vector** | |
| X | -2.73 | -2.89 | 0.93 | 0.60 | -1.66 |
| Y | -1.83 | -4.67 | -1.69 | -4.62 | 1.06 |
| Z | 0.12 | -0.84 | 0.14 | 0.67 | 1.16 |

The coordinates of the model set forth in the Tables II defines the relative relationship between the features. The coordinates are dependent upon the particular coordinate system used, and those skilled in the art will recognize that, although rotation and translation of these coordinates may change the specific values of these coordinates, they will in fact define the claimed model. The claimed model is intended to encompass any model, after optimal superposition of the models, comprising the identified features and having a root mean square of equivalent features of less than about 3.0 Å. More preferably, the claimed model encompasses any model comprising the identified features and having a root mean square of equivalent features of less than about 1.5 Å, and most preferably, less than 1.0 Å.

CERIUS² ™ pharmacophore models generated for SSRI compounds may be quite similar in terms of descriptor content. 3D-QSAR approaches, using, *e.g*., CATALYST™ and CERIUS² ™, may be employed to predict the molecules in the test set. The equations in a CERIUS² ™ model generally define the relative relationship between the descriptors and the CYP2D6 inhibitory potency values. Those skilled in the art will recognize that such equations may alter slightly depending on the nature of the algorithm used. The equations generated define the models of the invention, which is intended further to encompass any other model, after superposition of the models comprising the above-identified equations.

The pharmacophore models of the invention can be used to evaluate the inhibitory potency of a compound towards CYP2D6 and thereby identify those SSRI compounds which have potent CYP2D6 interactions or inhibitory activity. The compounds being evaluated may be designed *de novo* using the models of the invention, or alternatively, be a compound, *e.g.,* chosen from a library of compounds. Using the model of the invention and the methods of identification disclosed herein, one may predict the CYP2D6 inhibitory potency of a compound based upon its fit with the pharmacophore model of the invention. Further, one may even predict the relative degree of CYP2D6 inhibitory potency via the methods of the invention by calculation of the K_{i (apparent)} value for a compound.

After identifying an SSRI compound to be evaluated for CYP2D6 inhibitory potency, the three-dimensional structure of the compound is determined. This may already have been done if, *e.g.,* the compound is obtained from a structural database wherein three-dimensional x, y and z coordinates are used to define the compound. Alternatively, the three-dimensional structures of small molecules can be readily determined by methods known to those of skill in the art, including but not limited to, X-ray crystallography, nuclear magnetic resonance spectrometry, *etc.* The structures obtained from structural databases are usually the structures of compounds alone, uncomplexed with other molecules. If the three-dimensional structure is not known, one may use computer programs, including but not limited to, CATALYST™, to predict the three-dimensional structure of the compound. Three-dimensional conformers are generated from a starting structure using methods well known in the art such as, but not limited to, *e.g.,* the Best or Fast Conformational Analyses (Molecular Simulations, Inc., San Diego, California) with an energy set to a range of 0 to 50 Kcal/mol, preferably 0 to 35 Kcal/mole, and most preferably 0 to 10 Kcal/mole, and the maximum number of conformations set to 100, preferably 175, and most preferably 255. The pharmacophore model may be then compared to evaluated subject compounds, *i.e.,* SSRI antagonists, using tools to compare the structural features of each, such as, *e.g.,* COMPARE™ within the VIEW HYPOTHESIS™ workbench (Molecular Simulations, Inc., San Diego, California).

The degree of fit of a particular compound structure to the pharmacophore model is calculated by determining, using computer methods, if the compound possesses the chemical features of the model and if the features can adopt the necessary three-dimensional arrangement to fit the model. The modeling program will indicate those features in the model having a fit with the particular compound.

In preferred embodiments, the present invention encompasses compounds that are human SSRI compounds that do not possess significant inhibitory potency towards the CYP2D6 enzyme as identified by the above-described methods having a useful selectivity and specificity. Absolute values of receptor activity and potency can vary widely and may be readily determined by those skilled in the art based on the desired application of the antagonist and the situations in which CYP2D6 interactions are critical to the acceptability of the antagonist as an effective therapeutic agent. In more preferred embodiments, the inhibitory potency of the SSRI compound towards CYP2D6 will be represented by a K_{i (apparent)} value of 1 µM or greater; more preferably 10 µM or greater; and most preferably 100 µM or greater.

In another embodiment of the invention, a three-dimensional representation of the pharmacophore for the CYP2D6 inhibitory potency of SSRI compounds is created. Specifically, an SSRI compound is optimally superimposed on the pharmacophore model using computational methods well known to those of skill in the art as implemented in, *e.g*., CATALYST™ (Molecular Simulations, Inc., San Diego, California). A superposition of structures and the pharmacophore model is defined as a minimization of the root mean square distances between the centroids of the corresponding features of the molecule and the pharmacophore. A van der Waals surface is then calculated around the superimposed structures using a computer program such as CERIUS² ™ (Molecular Simulations, Inc., San Diego, California). Not being bound by any theory, the van der Waals surfaces of compounds with low K_{i (apparent)} values are thought to approximate the shape of the active site in the CYP2D6 enzyme, since these compounds will have the most complementary fit to that site. As noted above, the active site for CYP2D6 has been studied. *(see,* Strobl *et al*., *supra*). The active site volume of CYP2D6 can be utilized, like the pharmacophore models of the invention, to determine the extent of fit of SSRI compounds into the enzyme active site, and thereby the extent of inhibition of the enzyme by those compounds. SSRI antagonist compounds having a better fit into the active site will most likely be more potent inhibitors of the CYP2D6 enzyme, while compounds that, *e.g.,* have poorer fit to the active site, will be less likely to possess significant inhibitory potency towards the human CYP2D6 enzyme.

Fitting of a compound to the active site volume can be done in a number of different ways using computational methods well known in the art. Visual inspection and manual docking of compounds into the active site volume can be done using such programs as QUANTA (Molecular Simulations, Burlington, MA, 1992), SYBYL (Molecular Modeling Software, Tripos Associates, Inc., St. Louis, MO, 1992), AMBER (Weiner *et al*., *J. Am. Chem. Soc.,* **106:** 765-784, 1984), or CHARMM (Brooks *et al*., *J. Comp. Chem.,* **4:** 187-217, 1983). This modeling step may be followed by energy minimization using standard force fields, such as CHARMM or AMBER. Other more specialized modeling programs include GRID (Goodford *et al*., *J*. *Med. Chem., **28**:* 849-857, 1985), MCSS (Miranker & Karplus, *Function and Genetics,* **11:** 29-34, 1991), AUTODOCK (Goodsell & Olsen, *Proteins: Structure, Function and Genetics,* **8:** 195-202, 1990), and DOCK (Kuntz *et al*., *J. Mol. Biol*., **161**:269-288 (1982)). In addition, inhibitor compounds may be constructed *de novo* in an empty active site or in an active site including some portions of a known inhibitor using computer programs such as LUDI (Bohm, *J*. *Comp. Aid. Molec. Design,* **6:** 61-78, 1992), LEGEND (Nishibata & Itai, *Tetrahedron*, **47:** 8985, 1991), and LeapFrog (Tripos Associates, St. Louis, MO).

The SSRI compounds, which possess no significant CYP2D6 interactions or inhibitory potency, identified using the pharmacophore model and methods of the invention can be administered to a patient, either alone or in pharmaceutical compositions where they are mixed with a suitable carrier(s) or excipient(s) at doses to treat or ameliorate a variety of diseases, conditions and disorders. A therapeutically effective dose further refers to that amount of the compound sufficient to result in amelioration of the disease, condition or disorder.

The pharmaceutical compositions of the present invention comprise SSRI compounds that do not possess significant inhibitory potency towards CYP2D6, said compounds may have chiral centers and therefore exist in different enantiomeric forms. Further, the methods of treatment of the present invention comprise the administration of such compounds having chiral centers. Accordingly, this invention includes methods and pharmaceutical compositions, as described above, wherein the SSRI compounds, which do not possess significant inhibitory potency towards CYP2D6, employed are optical isomers, tautomers, stereoisomers or mixtures thereof.

The present invention also relates to pharmaceutical compositions comprising a pharmaceutically acceptable acid addition salt of an SSRI identified in accordance with the methods of the present invention. Further, the invention also relates to methods of treatment comprising the administration of such an acid addition salt to a subject in need thereof. The possible acids which are used to prepare a pharmaceutically acceptable acid addition salt of a basic SSRI employed in the methods of this invention are those which form non-toxic acid addition salts, i.e., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [*i*.*e*., 1,1'-methylene-bis-(2-hydroxy-3-naphthoate)] salts.

The present invention also relates to pharmaceutical compositions comprising a pharmaceutically acceptable base addition salt of an SSRI compound identified in accordance with the methods of the present invention. Further, the invention also relates to methods of treatment comprising the administration of such a base addition salt to a subject in need thereof. The chemical bases that may be used as reagents to prepare a pharmaceutically acceptable base salt of an SSRI compound identified in accordance with the methods of the invention are those that form non-toxic base salts with such compounds. Such non-toxic base salts include, but are not limited to those derived from such pharmacologically acceptable cations such as alkali metal cations *(e.g.,* potassium and sodium) and alkaline earth metal cations *(e.g.,* calcium and magnesium), ammonium or water-soluble amine addition salts such as N-methylglucamine-(meglumine), and the lower alkanolammonium and other base salts of pharmaceutically acceptable organic amines.

The subject invention also relates to pharmaceutical compositions and methods of treatment that employ isotopically-labeled compounds that are identical to the SSRI compounds generated in accordance with the methods of the present invention, but for the fact that one or more atoms are replaced by an atom having an atomic mass or mass number different from the atomic mass or mass number usually found in nature. Examples of isotopes that can be incorporated into the SSRI compounds that are employed in the pharmaceutical compositions and methods of the present invention include isotopes of hydrogen, carbon, nitrogen, oxygen, phosphorous, fluorine and chlorine, such as ²H, ³H, ¹¹C, ¹³C, ¹⁴C, ¹⁵N, ¹⁸O, ¹⁷O, ³¹P, ³²P, ³⁵S, ¹⁸F, and ³⁶Cl, respectively. The SSRI compounds employed in the pharmaceutical compositions and methods of the present invention, prodrugs thereof, and pharmaceutically acceptable salts of said compounds or of said prodrugs which contain the aforementioned isotopes and/or other isotopes are within the scope of this invention. Certain isotopically-labeled SSRI's, for example, those into which radioactive isotopes such as ³H and ¹⁴C are incorporated, are useful in drug and/or substrate tissue distribution assays. Tritiated, *i.e.,* ³H, and carbon-14, *i.e.,* ¹⁴C, isotopes are particularly preferred for their ease of preparation and detectability. Further, substitution with heavier isotopes such as deuterium, *i.e*., ²H, can afford certain therapeutic advantages resulting from greater metabolic stability, for example increased *in vivo* half-life or reduced dosage requirements and, hence, may be preferred in some circumstances.

This invention relates both to methods of treating diseases, conditions or disorders in which the SSRI, or the pharmaceutically acceptable salt of another therapeutically active agent, are administered together, as part of the same pharmaceutical composition, as well as to methods in which these two active agents are administered separately as part of an appropriate dose regimen designed to obtain the benefits of the combination therapy. The appropriate dose regimen, the amount of each dose administered, and specific intervals between doses of each active agent will depend upon the subject being treated, the emetogen and the severity of the condition. Generally, in carrying out the methods of this invention, the SSRI compound will be administered to an adult human in an amount ranging from about 0.01 to 250 mg per day, preferably about 0.07 to 21 mg per day, and the other pharmaceutically active agent or pharmaceutically acceptable salt thereof will be administered in an amount sufficient to ameliorate the condition being treated. Variations may nevertheless occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effect, provided that such larger doses are first divided into several small doses for administration throughout the day.

The SSRI's and their pharmaceutically acceptable salts that are employed in the pharmaceutical compositions and methods of this invention are hereinafter also referred to as "therapeutic agents." The therapeutic agents can be administered via either the oral or parenteral route. Compositions containing both an SSRI or a pharmaceutically acceptable salt thereof will generally be administered orally or parenterally daily, in single or divided doses, so that the total amount of each active agent administered falls within the above guidelines.

The therapeutic agents may be administered alone or in combination with pharmaceutically acceptable carriers or diluents by either of the routes previously indicated, and such administration may be carried out in single or multiple doses. More particularly, the novel therapeutic agents of this invention can be administered in a wide variety of different dosage forms, *i.e*., they may be combined with various pharmaceutically acceptable inert carriers in the form of tablets, capsules, lozenges, troches, hard candies, suppositories, aqueous suspensions, injectable solutions, elixirs, syrups, and the like. Such carriers include solid diluents or fillers, sterile aqueous media and various non-toxic organic solvents, *etc.* Moreover, oral pharmaceutical compositions can be suitably sweetened and/or flavored. In general, the therapeutic compounds of this invention, when administered separately (*i.e*., not in the same pharmaceutical composition) are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a therapeutic agent in either sesame or peanut oil or in aqueous propylene glycol may be employed. The aqueous solutions should be suitably buffered if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

As stated above, the SSRI may be formulated in a single pharmaceutical composition or alternatively in individual pharmaceutical compositions for simultaneous, separate or sequential use in accordance with the present invention.

Preferably the compositions according to the present invention are in unit dosage forms such as tablets, pills, capsules, powders, granules, solutions or suspensions, or suppositories, for oral, parenteral or rectal administration, by inhalation or insufflation or administration by transdermal patches or by buccal cavity absorption wafers.

For preparing solid compositions such as tablets, the principal active ingredient is mixed with a pharmaceutical carrier, *e.g.,* conventional tabletting ingredients such as corn starch, lactose, sucrose, sorbitol, talc, stearic acid, magnesium stearate, dicalcium phosphate or gums, and other pharmaceutical diluents, *e.g.,* water, to form a solid pre-formulation composition containing a homogeneous mixture of a compound of the present invention, or a non-toxic pharmaceutically acceptable salt thereof. When referring to these pre-formulation compositions as homogeneous, it is meant that the active ingredient is dispersed evenly throughout the composition so that the composition may be readily subdivided into equally effective unit dosage forms such as tablets, pills and capsules. This solid pre-formulation composition is then subdivided into unit dosage forms of the type described above containing from about 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac acetyl alcohol and cellulose acetate.

The liquid forms in which the novel compositions of the present invention may be incorporated for administration orally or by injection include aqueous solutions, suitably flavored syrups, aqueous or oil suspensions, and flavored emulsions with edible oils such as cottonseed oil, sesame oil, coconut oil, peanut oil or soybean oil, as well as elixirs and similar pharmaceutical vehicles. Suitable dispersing or suspending agents for aqueous suspensions include synthetic and natural gums such as tragacanth, acacia, alginate, dextran, sodium carboxymethyl cellulose, methylcellulose, polyvinyl-pyrrolidone or gelatin.

Preferred compositions for administration by injection include those comprising an SSRI, as the active ingredient, in association with a surface-active agent (or wetting agent or surfactant) or in the form of an emulsion (as a water-in-oil or oil-in-water emulsion).

Suitable surface-active agents include, in particular, non-ionic agents, such as polyoxyethylenesorbitans ( *e.g.,* Tween 20, 40, 60, 80 or 85) and other sorbitans (*e.g*., Span 20, 40, 60, 80 or 85). Compositions with a surface-active agent will conveniently comprise between 0.05 and 5% surface-active agent, and preferably between 0.1 and 2.5%. It will be appreciated that other ingredients may be added, for example mannitol or other pharmaceutically acceptable vehicles, if necessary.

Suitable emulsions may be prepared using commercially available fat emulsions, such as Intralipid, Liposyn, Infonutrol, Lipofundin and Lipiphysan. The active ingredient may be either dissolved in a pre-mixed emulsion composition or alternatively it may be dissolved in an oil *(e.g.,* soybean oil, safflower oil, cottonseed oil, sesame oil, corn oil or almond oil) and an emulsion formed upon mixing with a phospholipid (*e*.*g*., eggs phospholipids, soybean phospholipids or soybean lecithin) and water. It will be appreciated that other ingredients may be added, for example glycerol or glucose, to adjust the tonicity of the emulsion. Suitable emulsions will typically contain up to 20% oil, for example, between 5 and 20%. The fat emulsion will preferably comprise fat droplets between 0.1 and 1.0 µm, particularly 0.1 and 0.5 µm, and have a pH in the range of 5.5 to 8.0.

Compositions for inhalation or insufflation include solutions and suspensions in pharmaceutically acceptable, aqueous or organic solvents or mixtures thereof, and powders. The liquid or solid compositions may contain suitable pharmaceutically acceptable excipients as set out above. Preferably the compositions are administered by the oral or nasal respiratory route for local or systemic effect. Compositions in preferably sterile pharmaceutically acceptable solvents may be nebulized by use of inert gases. Nebulized solutions may be breathed directly from the nebulizing device or the nebulizing devise may be attached to a face mask, tent or intermittent positive pressure breathing machine. Solution, suspension, or powder compositions may be administered, preferably orally or nasally, from devices which deliver the formulation in an appropriate manner.

Compositions of the present invention may also be presented for administration in the form of transdermal patches using conventional technology. The compositions may also be administered via the buccal cavity using, for example, absorption wafers.

The present invention further provides a process for the preparation of a pharmaceutical composition comprising an SSRI compound, which process comprises bringing the SSRI compound into association with a pharmaceutically acceptable carrier or excipient.

When administered in combination, either as a single or as separate pharmaceutical composition(s), the SSRI compound and another pharmaceutically active agent are presented in a ratio which is consistent with the manifestation of the desired effect. A suitable dosage level for the SSRI compound is about 0.01 to 250 mg per day, preferably about 0.07 to 21 mg per day. The compounds may be administered on a regimen of up to 6 times per day, preferably 1 to 4 times per day. Other preferred embodiments include the delivery of the SSRI compound using an oral dosage form or by injection. Variations may nevertheless occur depending upon the subject being treated and its individual response to said medicament, as well as on the type of pharmaceutical formulation chosen, and the time period, and interval, at which such administration is carried out. In some instances, dosage levels below the lower limit of the aforesaid range may be more than adequate, while in other cases still larger doses may be employed without causing any harmful side effect, provided that such larger doses are first divided into several small doses for administration throughout the day.

It will be appreciated that the amount of an SSRI compound required for use in the treatment or prevention of a condition, disorder or disease will vary not only with the particular compounds or compositions selected but also with the route of administration, the nature of the condition being treated, and the age and condition of the patient, and will ultimately be at the discretion of the patient's physician or pharmacist.

The serotonin receptor binding affinities of the SSRI compounds of the present invention, *i.e.,* those identified by the methods of the invention, as such, can be determined using standard radioligand binding assays as described in the literature. For example, 5-HT_{1A} receptor binding affinities can be measured using the procedure of Hoyer *et al*. (*Brain Res*., *376,* 85 (1986)), and 5-HT_{1D} binding affinities can be measured using the procedure of Heuring and Peroutka (*J. Neurosci.,* 7, 894 (1987)); the contents of these documents are incorporated herein by reference.

*In vitro* binding activity at the 5-HT_{1D} receptor binding site is, for example, determined according to the following procedure. Bovine caudate tissue is homogenized and suspended in 20 volumes of a buffer containing 50 mM TRIS-HCl (tris[hydroxymethyl]aminomethane hydrochloride) at a pH of 7.7, following which the homogenate is centrifuged at 45,000g for 10 minutes. The resulting supernatant is discarded, and the pellet is resuspended in approximately 20 volumes of 50 mM TRIS-HCl buffer at pH 7.7; said suspension is pre-incubated for 15 minutes at 37°C, after which it is centrifuged again at 45,000G for 10 minutes. The resulting supernatant discarded, and the pellet (approximately 1 gram) is resuspended in 150 ml of a buffer of 15 mM TRIS-HCl containing 0.01 percent ascorbic acid, final pH 7.7, 10 µM pargyline and 4 mM calcium chloride (CaCl₂) - the suspension is kept on ice at least 30 minutes prior to use.

The inhibitor, control or vehicle is incubated according to the following procedure: to 50 µl of a 20 percent dimethylsulfoxide (DMSO)/80 percent distilled water solution is added 200 µl of tritiated 5-hydroxytryptamine (2 nM) in a buffer of 50 mM TRIS-HCl containing 0.01 percent ascorbic acid at pH 7.7, 10 µM pargyline, 4 mM calcium chloride, 100 nM of 8-hydroxy-DPAT (dipropylaminotetraline) and 100 nM of mesulergine. To this mixture is added 750 µl of bovine caudate tissue, and the resulting suspension is vortexed to ensure a homogenous suspension; the suspension is then incubated in a shaking water bath for 30 minutes at 25°C; after incubation is complete, the suspension is filtered using glass fiber filters (*e.g*., Whatman GF/B-filters). The pellet is washed three times with 4 ml of a buffer of 50 mM TRIS-HCl (pH 7.7), and is then placed in a scintillation vial with 5 ml of scintillation fluid (aquasol 2) and allowed to sit overnight. The percent inhibition is calculated for each dose of the compound, and an IC₅₀ value is then calculated from the percent inhibition values.

Binding affinities at the 5-HT_{1A} receptor is, for example, determined according to the following procedure. Rat brain cortex tissue is homogenized and divided into samples of 1 g lots and diluted with 10 volumes of 0.32 M sucrose solution. The suspension is then centrifuged at 900g for 10 minutes, the supernatant separated and recentrifuged at 70,000g for 15 minutes and the pellets are then collected and resuspended in 10 volumes of 15 mM TRIS-HCl (pH 7.5); the remaining supernatant is discarded. The resulting suspension is allowed to incubate for 15 minutes at 37°C, after which it is then centrifuged at 70,000g for 15 minutes and the supernatant discarded. The resulting tissue pellet is resuspended in a buffer of 50 mM TRIS-HCl (pH 7.7) containing 4 mM of calcium chloride and 0.01 percent ascorbic acid - this tissue suspension is stored at -70°C until ready for an experiment.

The tissue can be thawed immediately prior to use, diluted with 10 µM pargyline and kept on ice; tissue incubation is according to the following procedure. Fifty microliters of control, inhibitor, or vehicle (1 percent DMSO final concentration) is prepared at various dosages. To this solution is added 200µl of tritiated 8-hydroxy DPAT at a concentration of 1.5 nM in a buffer of 50 mM TRIS-HCl at pH 7.7, containing 4 mM calcium chloride, 0.01 percent ascorbic acid and pargyline. 750 µl of tissue is added, the resulting suspension is vortexed to ensure homogeneity, and is then incubated in a shaking water bath for 30 minutes at 37°C. The solution is filtered, and then washed twice with 4 ml of 10 mM TRIS-HCI at pH 7.5 containing 154 mM of sodium chloride.

Agonist and antagonist activities of compounds of formula (I) at the 5-HT_{1A} and 5-HT_{1D} receptors is, for example, determined using a single saturating concentration according to the following procedure. Male Hartley guinea pigs are decapitated and 5-HT_{1A} receptors are dissected out of the hippocampus, while 5-HT_{1D} receptors are obtained by slicing at 350 mm on a McIlwain tissue chopper and dissecting out the substantia nigra from the appropriate slices. The individual tissues are homogenized in a 5 mM HEPES buffer containing 1 mM EGTA (pH 7.5) using a hand-held glass-Teflon® homogenizer and centrifuged at 35,000g for 10 minutes at 4°C. The resulting pellets are resuspended in a 100 mM HEPES buffer containing 1 mM EGTA (pH 7.5), to a final protein concentration of 20 mg (hippocampus) or 5 mg (substantia nigra) of protein per tube; the following agents are added so that the reaction mix in each tube contains 2.0 mM MgCl₂, 0.5 mM ATP, 1.0 mM cAMP, 0.5 mM IBMX, 10 mM phosphocreatine, 0.31 mg/mL creatine phosphokinase, 100 µM GTP and 0.5-1 microcuries of [³²P]-ATP (30 Ci/mmol: NEG-003 - New England Nuclear). Incubation is initiated by the addition of tissue to siliconized microfuge tubes (in triplicate) at 30°C for 15 minutes. Each tube receives 20 µl tissue, 10 µl drug or buffer (at 10X final concentration), 10 µl of 32 nM agonist or buffer (at 10X final concentration), 20 µl forskolin (3 µM final concentration) and 40 µl of the preceding reaction mix. Incubation is terminated by the addition of 100 µl 2% SDS, 1.3 mM cAMP, 45 mM ATP solution containing 40,000 dpm [³H]-cAMP (30 Ci/mmol: NET-275 - New England Nuclear) to monitor the recovery of cAMP from the columns (the separation of [³²P]-ATP and [³²P]-cAMP is accomplished using the method of Salomon *et al*., *Analytical Biochemistry,* 1974, *58*, 541-548, the contents of which are incorporated herein by reference). Radioactivity is quantified by liquid scintillation counting. Maximal inhibition is defined by 10 µM (R)-8-OH-DPAT for 5-HT_{1A} receptors, and 320 nM 5-HT for 5-HT_{1D} receptors. Percent inhibitions by the test compounds are then calculated in relation to the inhibitory effect of (R)-8-OH-DPAT for 5-HT_{1A} receptors or 5-HT for 5-HT_{1D} receptors. The reversal of agonist-induced inhibition of forskolin-stimulated adenylate cyclase activity is calculated in relation to the 32 nM agonist effect.

The compounds of this invention are, for example, tested for *in vivo* activity for antagonism of 5-HT_{1D} agonist-induced hypothermia in guinea pigs according to the following procedure. Male Hartley guinea pigs from Charles River, weighing 250-275 grams on arrival and 300-600 grams at testing, serve as subjects in the experiment. The guinea pigs are housed under standard laboratory conditions on a 7 a.m. to 7 p.m. lighting schedule for at least seven days prior to experimentation. Food and water are available *ad libitum* until the time of testing. Compounds of formula (I) are administered, for example, as solutions in a volume of 1 ml/kg; the vehicle used is varied depending on compound solubility. Test compounds are typically administered either sixty minutes orally (p.o.) or 0 minutes subcutaneously (s.c.) prior to administration of a 5-HT_{1D} agonist, such as [3-(1-methylpyrrolidin-2-ylmethyl)-1H-indol-5-yl]-(3-nitropyridin-3-yl)-amine, which can be prepared as described in PCT publication WO93/111 06, published June 10, 1993 (the contents of which are incorporated herein by reference), and which is administered at a dose of 5.6 mg/kg, s.c.

Before a first temperature reading is taken, each guinea pig is placed in a clear plastic shoe box containing wood chips and a metal grid floor and allowed to acclimate to the surroundings for 30 minutes. Animals are then returned to the same shoe box after each temperature reading. Prior to each temperature measurement each animal is firmly held with one hand for a 30-second period. A digital thermometer with a small animal probe is used for temperature measurements. The probe is made of semi-flexible nylon with an epoxy tip. The temperature probe is inserted 6 cm. into the rectum and held there for 30 seconds or until a stable recording is obtained. Temperatures are then recorded.

In p.o. screening experiments, a "pre-drug" baseline temperature reading is made at -90 minutes, the test compound is given at -60 minutes and an additional -30 minute reading is taken. The 5-HT_{1D} agonist is then administered at 0 minutes and temperatures are taken 30, 60, 120 and 240 minutes later. In subcutaneous screening experiments, a pre-drug baseline temperature reading is made at -30 minutes. The test compound and 5-HT_{1D} agonists are given concurrently and temperatures are taken at 30, 60, 120 and 240 minutes later. Data are analyzed with two-way analysis of variants with repeated measures in Newman-Keuls post hoc analysis.

The serotonin 5-HT₁ agonist activity can be determined by *in vitro* receptor binding assay, as described for the 5-HT_{1A} receptor using rat cortex as the receptor source and [³H]-8-OH-DPAT as the radioligand [D. Hoyer *et al. Eur. J*. *Pharm*., *118*, 13 (1985)] and as described for the 5-HT_{1D} receptor using bovine caudate as the receptor source and [³H]serotonin as the radioligand [R.E. Heuring and S.J. Peroutka, *J*. *Neuroscience,* 7, 894 (1987)]; the contents of these documents are incorporated herein by reference.

The binding activity at the 5-HT_{2A} receptor is, for example, determined according to the following procedure. Male Sprague-Dawley rats are decapitated and their brains removed. Frontal cortices are dissected and homogenized in 50 mM Tris HCl buffer (pH 7.4 at 4°C) containing 2 mM MgCl2 using a Polytron homogenizer (setting 15,000 rpm). The homogenate is centrifuged for ten minutes at 40,000 x g (20,000 rpm in a Sorvall SS34 rotor). The supernatant was discarded and the pellet resuspended with the Polytron homogenizer in fresh ice-cold 50 mM TRIS HCl (pH 7.4 at 4°C) buffer containing 2 mM MgCl2 and centrifuged again. The final pellet was resuspended in 50 mM Tris HCl buffer (pH 7.7 at 22°C) for a final tissue concentration of 9 mgs wet weight tissue per mL buffer. Incubation is initiated by the addition of tissue to V-bottom polypropylene 96 well plates (in triplicate). Incubation is at 37°C for 15 minutes in a water bath. Each tube receives 200 µL tissue suspension, 25 µL ³H-ketanserin (0.4 nM final concentration), and 25 µL drug or buffer. Nonspecific binding is determined using 10 µM cinanserin. Incubation is ended by rapid filtration under vacuum through fire-treated Whatman GF/B glass fiber filters (presoaked in 0.5% polyethenylenimine (PEI) and dried) and rinsed with ice-cold 50 mM Tris HCl buffer (pH 7.7 at 4°C), setting 555 on a Skatron 96 well harvester. Filters are put into sample bags with 10 mL Betaplate scintillation fluid and allowed to sit 10 minutes before counting on a Betaplate scintillation counter (Wallac).

The binding activity at the α₁ receptor is, for example, determined according to the following procedure. Male Sprague-Dawley rats are decapitated and their brains removed. Cortices are dissected and homogenized in 50 mM Tris HCl buffer (pH 7.4 at 4°C) containing 2 mM MgCl2 using a Polytron homogenizer (setting 15,000 rpm). The homogenate is centrifuged for ten minutes at 40,000 x g (20,000 rpm in Sorvall SS34 rotor). The supernatant was discarded and the pellet resuspended with the Polytron homogenizer in fresh ice-cold 50 mM TRIS HCl (pH 7.4 at 4°C) buffer containing 2 mM MgCl2 and centrifuged again. The final pellet was resuspended in 50 mM Tris HCl buffer (pH 8.0 at 22°C) for a final tissue concentration of 12.5 mgs wet weight tissue per mL buffer. Incubation is initiated by the addition of tissue to V-bottom polypropylene 96 well plates (in triplicate). Incubation is at 25°C for 30 minutes on a shaker. Each tube receives 200 µL tissue suspension, 25 µL 3H-Prazosin (0.2 nM final concentration) and 25 µL drug or buffer. Nonspecific binding is determined using 10 µM phentolamine. Incubation is ended by rapid filtration under vacuum through fire-treated Whatman GF/B glass fiber filters (presoaked in 0.5% PEI and dried) and rinsed with ice-cold 50 mM Tris HCl buffer (pH 7.7 at 4°C), setting 555 on a Skatron 96 well harvester. Filters are put into sample bags with 10 mL Betaplate scintillation fluid and allowed to sit 10 minutes before counting on a Betaplate scintillation counter (Wallac).

The binding activity at the dopamine D₂ receptor is, for example, determined according to the following procedure. Male Sprague-Dawley rats are decapitated and their brains removed. Striata are dissected and homogenized in 50 mM Tris HCl buffer (pH 7.4 at 4°C) containing 2 mM MgCl₂ using a Polytron homogenizer (setting 15,000 rpm). The homogenate is centrifuged for ten minutes at 40,000 x g (20,000 rpm in a Sorvall SS34 rotor). The supernatant was discarded and the pellet resuspended with the Polytron in fresh ice-cold 50 mM Tris HCl (pH 7.4 at 4°C) containing 2 mM MgCl₂ buffer and centrifuged again. The final pellet was resuspended in 50 mM Tris HCl buffer containing 100 mM NaCl, 1 mM MgCl₂ (pH 7.4 at 37°C) for a final tissue concentration of 3 mg wet weight tissue per mL buffer. Incubation is initiated by the addition of tissue to V-bottom polypropylene 96 well plates (in duplicate or triplicate). Incubation is at 37°C for 15 minutes in a heated water bath. Each tube receives 200 µL tissue suspension, 25 µL ³H-spiperone (0.2 nM final concentration) and 25 µL drug or buffer. Nonspecific binding is determined using 10 µM (+)-butaclamol. Incubation is ended by rapid filtration under vacuum through fire-treated Whatman GF/B glass fiber filters (presoaked in 0.5% PEI and dried) and rinsed with ice-cold 50 mM Tris HCl buffer (pH 7.7 at 4°C), setting 555 on the Skatron 96 well harvester (15 sec wash). Filters are dried, put into sample bags with 10 mL Betaplate scintillation fluid and counted on a Betaplate scintillation counter (EG&G/Wallac).

The neurotransmitter uptake activity in rat synaptosomes or HEK-293 cells transfected with the human serotonin, dopamine or norepinephrine transporter is, for example, determined according to the following procedure. For rat synaptosomes preparation, male Sprague Dawley rats are decapitated and the brains removed. The cortex, hippocampi and corpus striata are dissected out and placed in ice cold sucrose buffer, 1 gram in 20 mls (320 mM sucrose containing 1mg/ml glucose, 0.1mM EDTA and brought up to pH 7.4 with Tris base). The tissues are homogenized in a glass homogenizing tube with a teflon pestle at 350 RPMS using a Potters homogenizer. The homogenate is centrifuged at 1000 x g for 10 min, at 4 C. The resulting supernatant is re-centrifuged at 17,000 x g for 20 min, at 4 C. The final pellet is then resuspended in an appropriate volume of sucrose buffer that yielded less than 10% uptake.

For cell preparation, HEK-293 cells transfected with the human serotonin (5-HT), norepinephrine (NE) or dopamine (DA) transporter were grown in DMEM (Gibco) supplemented with 10% dialyzed FBS (Gibco), 2 mM L-glutamine and 250 µg/ml G418 for the 5-HT and NE transporter or 2µg/ml puromycin for the DA transporter, for selection pressure. The cells were grown in Gibco triple flasks, harvested with PBS and diluted to an appropriate amount to yield less than 10 % uptake.

For the neurotransmitter uptake assay, the uptake assays were conducted in glass tubes containing 50 µL of solvent, inhibitor or 10µM sertraline, desipramine or nomifensine for the 5-HT, NE or DA assay nonspecific uptake, respectively. Each tube contained 400 *µ*L of [³H]5-HT (5 nM final), [³H]NE (20 nM final) or [³H]DA (5 nM final) made up in modified Krebs containing 100 µM pargyline and glucose (1mg/ml). The tubes were placed on ice, 50 µL of synaptosomes or cells was added to each tube. The tubes were then incubated at 37 C for the 7 min (5-HT, DA) or 10 min (NE). The incubation was terminated by filtration (GF/B filters), using a 96 well Brandel Cell Harvester, the filters were washed with modified Krebs buffer and either counted in a liquid scintillation counter or in a LKB Beta Plate counter.

Compounds prepared as working examples of the present invention and tested in accordance with the foregoing methods showed good binding activity in the range of more than 50% inhibition at <50 (fifty) nm concentration in the serotonin reuptake assay and binding assays for 5-HT_{2A} serotonin receptor while having an affinity of >100 (one hundred) nm at the dopamine D2 receptor, 5-HT_{1A} serotonin, 5-HT_{1D} or α₁ adrenergic receptor.

The present invention is illustrated by the following examples. It will be understood, however, that the invention is not limited to the specific details of these examples.

### EXAMPLES

### Example 1

### Molecular Modeling with CATALYST™

Computational molecular modeling studies were carried out using a Silicon Graphics Octane workstation and based on a methodology previously described for CYP2D6 and other CYPs (Ekins *et al*., *Pharmacogenetics*, **9:** 477-489, 1999; Ekins *et al*., *J*. *Pharmacol. & Exp. Ther*., **288:** 21-29, 1999; Ekins *et al*., *J. Pharmacol. & Exp. Ther*., **290:** 429-438, 1999; Ekins *et al*., *J. Pharmacol. & Exp. Ther*., **291:** 424-433, 1999). The three-dimensional structures for a training set of 14 amphetamine analogs, which were shown to bind competitively to the active site of CYP2D6 (Wu *et al*., *supra*; (*d*,*l*)-2-methoxy-4,5-methylenedioxyamphetamine; (*d*,*l*)-3,4-methylene-dioxymethamphetamine; (*d*,*l*)-3,4-methylenedioxyamphetamine; (*d*,*l*)-3-methoxy-4,5-methylenedioxyamphetamine; (*d*,*l*)-2-methoxyamphetamine; (*d*,*l*)-3-methoxy-amphetamine; (*d*,*l*)-4-methoxyamphetamine; (+)-methamphetamine; (+)-amphetamine; (*d*,*l*)-2,4,6-trimethoxyamphetamine; (*d*,*l*)-4-hydroxymethamphetamine; (*d*,*l*)-3,4,5-trimethoxyamphetamine; (+)-4-hydroxyamphetamine; and (*d*,*l*)-cathinone), were built interactively using CATALYST™ version 4.0 (Molecular Simulations, San Diego, CA). Multiple conformers were generated. The number of conformers generated for each inhibitor was limited to a maximum of 255 with an energy range of 20 Kcal/mol.

Ten hypotheses (pharmacophore test models) were generated using these conformers for each of the molecules using the following features: hydrogen bond acceptor, hydrogen bond donor, hydrophobic and ring aromatic as previously described (Ekins *et al*., *Pharmacogenetics*, **9:** 477-489, 1999) and K_{i (apparent)} values were generated. After assessing all 10 hypotheses (pharmacophore test models) generated for each data set, the lowest cost hypothesis (best pharmacophore test model) was considered the best.

The goodness of the structure activity correlation was estimated by means of the regression value (r). CATALYST**™** also calculated the total cost (goodness of fit) of the generated pharmacophores from the deviation between the estimated activity and the observed activity, combined with the complexity of the pharmacophore test model or hypothesis *(i.e.,* the number of pharmacophore features). A null hypothesis (null test model) is additionally calculated which presumes that there is no relationship in the data and that experimental activities are normally distributed about their mean. Hence the greater the difference between the cost (goodness of fit) of the generated hypothesis (pharmacophore test model) and the cost of the null hypothesis (null test model), the less likely it is that the test model reflects a chance correlation. This criteria was then used as an assessment of the pharmacophore model selected.

Using the training set consisting of 14 molecules, the resulting lowest cost pharmacophore yielded 3 features (Figure 1) necessary for inhibition of CYP2D6. The pharmacophore consists of 1 hydrogen bond acceptor, 1 hydrogen bond donor and 1 hydrophobic feature, all of which are within 4 Å of each other. The correlation for the best hypothesis (pharmacophore test model) generated an estimated versus observed K_{i (apparent)} value of r=0.87 which is comparable with previously generated CYP2D6 K_{i (apparent)} pharmacophores (Ekins *et al*., *Pharmacogenetics*, **9:** 477-489, 1999). The pharmacophore also possessed a total cost of 63.6 units as compared to the null hypothesis (null test model) of 73.2 units. Small cost differences between the observed pharmacophore and the null pharmacophore test model do not appear to hinder the predictive nature of such pharmacophores (Ekins *et* al., *Pharmacogenetics*, **9:** 477-489, 1999; Ekins *et al*., *J. Pharmacol.* Exp. *Ther.,* **288:** 21-29, 1999). This difference suggests the reasonableness of the pharmacophore test model.

### Example 2

### CATALYST ™ CYP2D6 Pharmacophore Validation Using A Test Set of SSRI's.

A set of 12 published SSRI's with known K_{i (apparent)} values (Table I) were then used as a test set to determine the predictive ability of the pharmacophore model, with K_{i (apparent)} values predicted for each. This is a valid test set for the pharmacophore model as each of the members of the set were excluded from the pharmacophore model.

The structures of these molecules were generated as described in Example 2 and multiple conformers were used for fitting to the resultant training set pharmacophore using the best fit function previously described (Ekins *et al., Pharmacogenetics*, **9:** 477-489, 1999). The best fit option was found to give the closest K_{i (apparent)} values and was therefore used to fit the complete test set of 12 SSRI's to the pharmacophore (Table I). The best fit function refers to the method of finding the optimum fit of the inhibitor to the pharmacophore model among all the conformers of the molecule without performing an energy minimization on the conformers of the molecule. (CATALYST™ tutorials release 3.0, Molecular Simulations, Inc., San Diego, CA.)

The predictions for the test set were then compared with their mean observed K_{i (apparent)} values obtained from the literature. Predictions within one log unit of the observed K_{i (apparent)} value were considered a valid prediction of fit. Because the variability of literature generated kinetic parameters is quite wide, the mean value was used as the observed K_{i (apparent)} when appropriate. Accordingly, the observed Kᵢ ₍ₐₚₚₐᵣₑₙₜ₎ in these cases were: sertraline, 6.52 µM; fluoxetine, 1.07 µM; norfluoxetine, 1.07 µM; citalopram, 10.37 µM; desmethylcitalopram, 3.65 µM; paroxetine, 0.19 µM; and fluvoxamine, 8.18 µM.

The pharmacophore model from Example 2 correctly predicted 10 of 12 SSRI's using a 1 log unit residual cutoff as previously described (Ekins *et al*., *J*. *Pharmacol*. *Exp. Ther*., **288:** 21-29, 1999), with only fluvoxamine and citalopram failing. Some of the deviation in the comparisons is dependent on the mean of the published data which in most cases is quite variable, *e.g.,* fluoxetine, norfluoxetine, sertraline, paroxetine and fluvoxamine K_{i (apparent)} values vary by > 1 log unit (Table I). Compared to the prediction of the 6 SSRI's used with previous pharmacophores in which paroxetine and desmethylcitalopram were poorly predicted (Ekins *et al., Pharmacogenetics*, **9:** 477-489, 1999), this new model appears to predict SSRI's very well. In addition, it is able to distinguish correctly the rank order for stereoisomers of fluoxetine and norfluoxetine as obtained *in vitro* (Stevens *et al*., *supra).*

### Example 3

### Modeling with CERIUS² ™

The 14 membered training set of amphetamines of Wu *et al*., *supra,* are aligned in CATALYST™ on the best hypothesis (pharmacophore test model) then imported into CERIUS² ™. Default two- and three-dimensional descriptors are generated in the 3D-QSAR functionality. Activity values are log transformed and added to the study table. More three-dimensional descriptors may be selected including HOMO, LUMO, Jurs and Shadow indices. An equation is generated using the genetic function approximation (GFA) to select descriptors, which relates to the CYP2D6 log inhibitory activity.

### Example 4

### CERIUS² ™ Model Validation Using a Test Set of K_{i (apparent)} Values

The descriptors found to explain activity are generated for the test set of 12 compounds in order to predict a CYP2D6 inhibitory potency value using the equation produced in CERIUS² ™. These predictions are compared with the *in vitro* observed values to determine how predictive the CERIUS² ™ model was using a 1 log unit cutoff and accepting that IC₅₀ values less than 1 µM or a K_{i (apparent)} values less than 1 µM likely represent active compounds in terms of CYP2D6 inhibition.

The set of 14 amphetamine compounds are used to create training sets for 3D-QSAR models using CERIUS² ™. Using the descriptors for all 14 molecules and the GFA method enables selection of the best equation to described the CYP2D6 inhibition.

The present invention is not to be limited in scope by the specific embodiments disclosed in the examples which are intended as illustrations of a few aspects of the invention and any embodiments which are functionally equivalent are within the scope of this invention. Further, various modifications of the invention in addition to those shown and described herein will become apparent to those skilled in the art and are intended to fall within the scope of the claims below.

## Claims

1. A method of generating a pharmacophore model for the CYP2D6 inhibitory potency of selective serotonin reuptake inhibitor compounds comprising the steps of
(i) generating a set of three-dimensional conformers for each of the compounds in a training set comprising five or more selective serotonin reuptake inhibitor compounds;
(ii) correlating each of the compounds of said training set with an observed value for CYP2D6 inhibitory potency;
(iii) generating from the conformers of step (i) a set of one or more pharmacophore test models, each said pharmacophore test model comprising three or more of the CYP2D6 enzyme active site features selected from the group consisting of the hydrogen bond donor feature, the hydrogen bond acceptor feature, the hydrophobic region feature, the ionizable region feature and the ring aromatic feature, arranged in three-dimensional space;
(iv) calculating the CYP2D6 inhibitory potency for each conformer generated in step (i) towards each of the pharmacophore test models generated in step (iii);
(v) calculating the total cost for each pharmacophore test model; and
(vi) choosing the lowest cost pharmacophore test model as the pharmacophore model.

2. The method of claim 1 wherein the steps are carried out using a molecular modeling software.

3. The method of claim 1 wherein the steps are carried out with the molecular modeling software CATALYST™, version 4.

4. The method of claim 1 wherein the training set of selective serotonin reuptake inhibitor compounds are chosen from selective serotonin reuptake inhibitor compounds with observed CYP2D6 K_{i (apparent)} values spanning at least three orders of magnitude.

5. The method of claim 4 wherein the observed CYP2D6 K_{i (apparent)} values vary from 0.1 µM to 100 µM.

6. The method of claim 1 wherein the training set of step (i) contains one or more compounds selected from the group consisting of: (*d*,*l*)-2-methoxy-4,5-methylenedioxyamphetamine; (*d*,*l*)-3,4-methylenedioxymethamphetamine; (*d*,*l*)-3,4-methylenedioxyamphetamine; (*d*,*l*)-3-methoxy-4,5-methylenedioxyamphetamine; (*d*,*l*)-2-methoxyamphetamine; (*d*,*l*)-3-methoxyamphetamine; (*d*,*l*)-4-methoxy-amphetamine; (+)-methamphetamine; (+)-amphetamine; (*d*,*l*)-2,4,6-trimethoxy-amphetamine; (*d*,*l*)-4-hydroxymethamphetamine; (*d*,*l*)-3,4,5-trimethoxyamphetamine; (+)-4-hydroxyamphetamine; and (*d*,*l*)-cathinone.

7. A method for screening a selective serotonin reuptake inhibitor compound for CYP2D6 inhibitory potency comprising the steps of:
(i) finding the optimum fit of the selective serotonin reuptake inhibitor compound to the pharmacophore model of claim 1; and
(ii) calculating a CYP2D6 inhibitory potency value for the selective serotonin reuptake inhibitor compound.

8. The method of claim 7 wherein finding the optimum fit in step (i) is carried out via the use of a fast-fit algorithm, a principle component analysis, a partial least squares technique, a linear regression technique or a non-linear regression technique.

9. A method of generating a pharmacophore model for the CYP2D6 inhibitory potency of selective serotonin reuptake inhibitor compounds comprising the steps of
(i) correlating the chemical features of the conformers of the compounds in a training set of selective serotonin reuptake inhibitor compounds with a set of two-and/or three-dimensional descriptors for the active site of the CYP2D6 enzyme; and
(ii) generating an equation relating the observed CYP2D6 inhibitory potency of said selective serotonin reuptake inhibitor compounds to a set of generated two-and/or three dimensional descriptors for the selective serotonin reuptake inhibitor compound.

10. The method of claim 9 wherein the steps are carried out using the set of two- and/or three-dimensional descriptors for selective serotonin reuptake inhibitor compounds chosen from the 3D-QSAR functionality and the genetic function approximation equation of the CERIUS² ™ software program.

11. The method of claim 9 wherein the CYP2D6 inhibitory potency is a value determined by *in vitro* of the inhibition of the CYP2D6 enzyme reaction with bufuralol, said value selected from the group consisting of the IC₅₀ value, the % inhibition value or the K_{i (apparent)} value.

12. A method for determining the CYP2D6 inhibitory potency of an selective serotonin reuptake inhibitor compound comprising the steps of
(i) generating the two- and/or three-dimensional descriptors for said selective serotonin reuptake inhibitor compound;
(ii) inputting said three-dimensional descriptors into an equation relating the observed CYP2D6 inhibitory activity of a set of selective serotonin reuptake inhibitor compounds to a set of three-dimensional descriptors generated for those selective serotonin reuptake inhibitor compounds; and
(iii) solving said equation for the CYP2D6 inhibitory activity of the selective serotonin reuptake inhibitor compound corresponding to the generated three-dimensional descriptors of step (i).

13. The method of claim 12 wherein steps (i) through (iii) are carried out using a CERIUS² ™ software program.

14. A pharmacophore model for the CYP2D6 inhibitory potency of selective serotonin reuptake inhibitor compounds generated in accordance with the method of claim 1 or 9.

15. A pharmacophore model for the CYP2D6 inhibitory potency of selective serotonin reuptake inhibitor compounds according to claim 14 comprising 1 hydrogen bond acceptor, 1 hydrophobic feature and 1 hydrogen bond donor.

16. A pharmacophore model for the CYP2D6 inhibitory potency of selective serotonin reuptake inhibitor compounds according to claim 15 comprising the following centroids and vectors:
| **Coordinates** | **Hydrogen Bond Acceptor** | | **Hydrogen Bond Donor** | | **Hydrophobic** |
|---|---|---|---|---|---|
| | | **Vector** | | **Vector** | |
| X | -2.73 | -2.89 | 0.93 | 0.60 | -1.66 |
| Y | -1.83 | -4.67 | -1.69 | -4.62 | 1.06 |
| Z | 0.12 | -0.84 | 0.14 | 0.67 | 1.16 |

17. A method for the identification of a selective serotonin reuptake inhibitor compound which does not possess significant inhibitory potency towards CYP2D6 comprising the steps of
(i) generating two- and/or three-dimensional descriptors for a selective serotonin reuptake inhibitor compound;
(ii) inputting said two- and/or three-dimensional descriptors for the selective serotonin reuptake inhibitor compound into the equation of claim 9;
(iii) solving said equation for the inhibitory activity of the selective serotonin reuptake inhibitor compound corresponding to the generated two- and/or three-dimensional descriptors of step (i); and
(iv) designating the compound as not being a significant inhibitor of CYP2D6 activity if the calculated K_{i (apparent)} value is greater than 1 µM.

18. A method according to claim 17 wherein the calculated K_{i (apparent)} value of step (iv) is greater than 100 µM.

19. A selective serotonin reuptake inhibitor compound which does not possess significant inhibitory potency towards CYP2D6 identified by the method of claim 17.

20. A method of designing *de novo* compounds that are selective serotonin reuptake inhibitor compounds that do not possess significant inhibitory potency towards CYP2D6 comprising the steps of
(i) correlating the two- and/or three-dimensional descriptors for a pharmacophore model for selective serotonin reuptake inhibitor compounds that possess significant inhibitory potency towards CYP2D6 with randomly generated molecules having chemical features corresponding to said descriptors; and
(ii) choosing a generated molecule with a CYP2D6 K_{i (apparent)} of 1 µM or greater.

21. A method of designing *de novo* selective serotonin reuptake inhibitor compounds which have selective inhibitory potency towards CYP2D6 comprising the steps of
(i) choosing a target degree of CYP2D6 inhibitory potency;
(ii) generating a set of two- and/or three-dimensional descriptors for a pharmacophore model for selective serotonin reuptake inhibitor compounds that possess significant inhibitory potency towards CYP2D6 corresponding to the inhibitory potency of step (i); and
(iii) correlating said descriptors of step (ii) with compounds having chemical features corresponding to said descriptors.

22. The method according to claim 21, wherein the inhibitory potency of step (i) is a CYP2D6 K_{i (apparent)} of 10 µM or greater.

23. A pharmaceutical composition comprising a selective serotonin reuptake inhibitor compound, which does not possess significant inhibitory potency towards CYP2D6, according to claim 19.

24. Use of a pharmaceutical composition according to claim 23 in the manufacture of a medicament for the treatment of a condition, disorder or disease for which a selective serotonin reuptake inhibitor compound is therapeutically useful.

25. A computer-readable medium having stored thereon a pharmacophore model for selective serotonin reuptake inhibitor compounds that possess significant inhibitory potency towards CYP2D6 generated in accordance with the method of claim 1 or claim 9.

26. A computer comprising the computer-readable medium according to claim 25.

27. A computer when operated by the computer-readable medium according to claim 25.
